# EUROPEAN PATENT SPECIFICATION

(11) **EP 2 014 670 B1**
(45) Date of publication and mention of the grant of the patent: **14.11.2012**
(21) Application number: 08104378.8
(22) Date of filing: 11.06.2008
(51) Int. Cl.: C07H 15/14, C07H 1/08, A61K 31/7028, A61P 35/00

(54) **Production of Glucosinolates from Agricultural By-Products and Waste**
Herstellung von Glucosinolaten aus landwirtschaftlichen Nebenprodukten und Abfall
Production de glucosinolates à partir de sous-produits agricoles et de déchets

(30) Priority: 12.06.2007 US 761883
(43) Date of publication of application: 14.01.2009
(73) Proprietor: Kraft Foods Global Brands LLC, Northfield, IL 60093 (US)
(72) Inventor: West, Leslie George, Winnetka, IL 60093 (US); Pomerleau, Theresa, Buffalo Grove, IL 60089 (US); Matusheski, Nathan V., Gurnee, IL 60031 (US); Ludwig, Cathy Jean, Grayslake, IL 60030 (US); Hestekin, Jamie, Fayetteville, AZ 72703 (US); Crowley, Colin, Wheeling, IL 60090 (US); Kim, Nam-Cheol, Deerfield, IL 60015 (US)
(74) Representative: Smaggasgale, Gillian Helen

(56) References cited:
- EP-A- 1 752 052
- A.B. HANLEY ET AL.: "Improved isolation of glucobrassicin and other glucosinolates" J. SCI. FOOD AGRIC., vol. 34, 1983, pages 869-873, XP002500771
- ROCHFORT ET AL: "The isolation and purification of glucoraphanin from broccoli seeds by solid phase extraction and preparative high performance liquid chromatography" JOURNAL OF CHROMATOGRAPHY, ELSEVIER SCIENCE PUBLISHERS B.V. AMSTERDAM, NL, vol. 1120, no. 1-2, 7 July 2006 (2006-07-07), pages 205-210, XP005504318 ISSN: 0021-9673
- FAHEY J W ET AL: "Separation and purification of glucosinolates from crude plant homogenates by high-speed counter-current chromatography" JOURNAL OF CHROMATOGRAPHY, ELSEVIER SCIENCE PUBLISHERS B.V. AMSTERDAM, NL, vol. 996, no. 1-2, 9 May 2003 (2003-05-09), pages 85-93, XP004427344 ISSN: 0021-9673
- FAHEY J W ET AL: "The chemical diversity and distribution of glucosinolates and isothiocyanates among plants" PHYTOCHEMISTRY, PERGAMON PRESS, GB, vol. 56, no. 1, 1 January 2001 (2001-01-01), pages 5-51, XP004291754 ISSN: 0031-9422
- SONG LIJIANG ET AL: "Purification of major glucosinolates from Brassicaceae seeds and preparation of isothiocyanate and amine metabolites" JOURNAL OF THE SCIENCE OF FOOD AND AGRICULTURE, WILEY & SONS, CHICHESTER, GB, vol. 86, no. 8, 1 June 2006 (2006-06-01), pages 1271-1280, XP002433552 ISSN: 0022-5142
- TROYER J K ET AL: "Analysis of glucosinolates from broccoli and other cruciferous vegetables by hydrophilic interaction liquid chromatography" JOURNAL OF CHROMATOGRAPHY, ELSEVIER SCIENCE PUBLISHERS B.V. AMSTERDAM, NL, vol. 919, no. 2, 15 June 2001 (2001-06-15), pages 299-304, XP004248013 ISSN: 0021-9673
- A.M. SZMIGIELSKA, J.J. SCHOENAU: "Use of anion-exchange membrane extraction for the high-performance liquid chromatographic analysis of mustard seed glucosinolates", J. AGRIC. FOOD. CHEM., vol. 48, 2000, pages 5190-5194,

## Description

The present invention relates to a process for producing glucosinolates, particularly glucoraphanin, from agricultural by-products and waste. Glucosinolates are chemoprotective precursor compounds. The glucosinolates produced by the method of the invention may be incorporated into a variety of food products, pharmaceuticals, health supplements, and related products.

### BACKGROUND

It is generally agreed that diet plays a large role in controlling the risk of developing cancers and that increased consumption of fruits and vegetables may reduce cancer incidences in humans. The presence of certain minor chemical components in plants may provide major protection mechanisms when delivered to mammalian cells. Moreover, providing pharmaceuticals, nutritional supplements, or foods fortified or supplemented with cancer-fighting chemical components derived from plants may provide additional health benefits. An important trend in the U.S. food industry is to promote health conscious food products.

Glucosinolates (*β*-thioglucoside-*N*-hydroxysulfates) are found in dicotyledenous plants and many plants of the order *Capparales.* Glucosinolates are found in the *Brasicaceae, Resedaceae,* and *Capparaceae* families but most commonly in the *Brassicaceae* family. Over one hundred glucosinolates have been characterized. Glucosinolates are sulfur-containing, water-soluble, anionic compounds of the general structure: 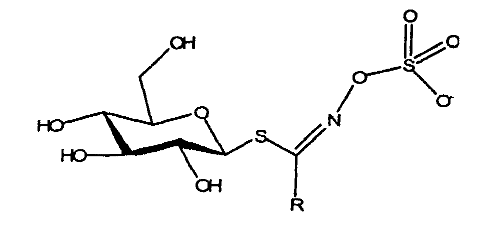 Glucosinolates include an R-group derived from amino acids and a thioglucosidic link to the carbon of a sulphonated oxime. The sulfate group imparts strongly acidic properties to glucosinolates.

Certain glucosinolates, particularly glucoraphanin (also known as sulforaphane glucosinolate or 4-methylsulfinyibutyl glucosinolate), are phytochemical precursors to potent chemoprotectants, such as sulforaphane. Glucosinolates and beta-thioglucosidase enzymes co-exist in glucosinolate-containing plants but are physically segregated until the cellular structure of the plant material is disrupted, such as by chewing, cutting, crushing, freeze-thawing, thermal treatment, or the like. Upon disruption of the cellular structure, the thioglucosidic bonds of the glucosinolates are hydrolyzed by beta-thioglucosidases, particularly myrosinase, into unstable glucosinolate aglycones, which undergo spontaneous rearrangement into potent chemoprotectants called isothiocyanates and other compounds. Isothiocyanates are biologically active and have high chemical reactivity. Isothiocyanates, particularly sulforaphane, appear to trigger carcinogen detoxification mechanisms when delivered to mammalian cells.

In addition to reducing the risk of certain cancers, glucoraphanin, through its bioactive conversion product sulforaphane, has recently been shown effective in destroying organisms responsible for causing stomach ulcers and may provide novel approaches for reducing the risk of developing cardiovascular and ocular diseases. Efforts are being undertaken to gain approval for making label claims on food products either naturally high in these agents or for foods containing added crucifer chemoprotectants. Products containing chemoprotectant additives, although without such label claims, are already on the market.

The production of glucosinolates, particularly glucoraphanin, is problematic because of their high cost. Generally, the best source of glucoraphanin has been expensive specialty broccoli cultivars. The considerable health potential of glucosinolates has not been realized due to the high cost of sourcing glucoraphanin.

Prior attempts have been made to obtain glucosinolates from plant materials. For example, ion-exchange columns and high-performance liquid chromatography (HPLC) have been used to isolate various glucosinolates. Bjerg, B. and S⌀rensen, H., Isolation of Intact Glucosinolates by Column Chromatography and Determination of Their Purity, in GLUCOSINOLATES IN RAPESEEDS: ANALYTICAL ASPECTS 59-75 (J-P. Wathelet ed., 1987). Fahey, J., et al., "The chemical diversity and distribution of glucosinolates and isothiocyanates among plants," Phytochemistry. 56: 5-51 (2001). Processes such as these cannot tolerate crude samples and require glucosinolate extracts that are clarified by filtration or centrifugation before processing in the columns.

In J. Agric. Food chem. 2000, 48, 5190-5194, a method for the extraction of glucosinolates using an anion-exchange membrane is described.

There remains a need for a more cost-effective process, which is efficient for obtaining glucosinolates, particularly glucoraphanin, from relatively crude starting materials. The present invention fulfills these, as well as other needs, as will be apparent from the following description of embodiments of the present invention.

### SUMMARY

The present invention is directed to a cost-effective process for obtaining glucosinolates, particularly glucoraphanin, from agricultural by-products and waste. Post-harvest and discarded plant materials, including non-saleable plant materials, make effective and inexpensive starting materials for the process of the invention.

The present method represents a significant advancement over methods recently presented in U.S. Patent Application Serial Nos. 11/199,752, filed August 9, 2005, 11/617,934, filed December 29, 2006, and 11/761,843 filed June 12, 2007, also owned by the same assignee of the present invention.

Cruciferous vegetables have been identified as a good source of chemoprotectant precursor phytochemicals. Cruciferous vegetables include, but are not limited to, broccoli, kale, collard, curly kale, marrowstem kale, thousand head kale, Chinese kale, cauliflower, Portuguese kale, brussel sprouts, kohlrabi, Jersey kale, Chinese broccoli, rutabaga, mustard, daikon, horseradish, cabbage, savoy cabbage, Chinese cabbage, napa cabbage, broccoli rabe, arugula, watercress, cress, turnip, borecole, radish, and the like. In a preferred aspect, broccoli plant materials are utilized.

Generally, when post-harvest plant materials are used in the method of the invention, the plant materials should be processed relatively quickly after harvesting. The plant materials may be chopped or cut but should not be crushed or puréed until immediately before use in the process of the invention in order to minimize damage to cellular structures. After the plant materials are cut or otherwise damaged during harvesting, the cell wall structures of the plant materials breaks down, thus bringing glucosinolates into contact with the enzymes that convert glucosinolates into Isothiocyanates. Therefore, damage to the glucosinolate-containing plant materials should be minimized prior to utilizing the plant materials in the method of the invention. In order to prolong the period of time the plant materials can be used post-harvest without deleterious impact on glucosinolate content, the glucosinolate-containing plant materials can be preserved by any conventional means, such as by freezing or drying, such as by air drying, freeze drying, vacuum drying, drum drying, spray drying, or the like. Generally, however, it is preferred that the plant material be used as soon as possible after harvesting.

Surprisingly, the process of the invention is effective for relatively crude starting materials. For example, the plant materials do not need to be washed or filtered to remove soil or other debris before use. The method of the invention generally comprises (1) providing a mixture of glucosinolate-containing plant material and liquid, (2) heating the mixture to inactivate enzymes in the plant material (3) contacting the heat inactivated mixture with an anion exchange membrane for 4 to 24 hours and at a temperature of 2°C to 7°C whereby at least a portion of the glucosinolates are absorbed onto the anion exchange membrane, and (4) releasing the glucosinolates from the anion exchange membrane. The method may further comprise removing salt from the extracted glucosinolates.

In another aspect of the invention, the method comprises the following steps: (1) providing a mixture of glucosinolate-containing plant material and liquid; (2) heating the mixture to inactivate enzymes in the plant material; (3) contacting the heat inactivated mixture with an anion exchange membrane for 4 to 24 hours at a temperature of 2°C to 7°C, whereby at least a portion of the glucosinolates are absorbed onto the anion exchange membrane; (4) releasing the glucosinolates from the anion exchange membrane; (5) contacting the heat inactivated mixture with an anion exchange membrane whereby at least a portion of the glucosinolates are absorbed onto the anion exchange membrane; and (6) releasing the glucosinolates form the anion exchange membrane. The method may further comprise removing salt from the extracted glucosinolates.

Generally, different types of glucosinolates will bind to the membrane and the choice of plant material will dictate the final composition. Therefore, it is desirable to select plant materials containing higher levels of the glucosinolate desired to be extracted. Preferably, plant materials containing glucoraphenin or glucoraphanin are selected. More preferably, plant materials containing glucoraphanin are selected.

The glucosinolate extract produced by the method of the invention may be incorporated into food products, pharmaceuticals, and health supplements. The glucosinolate extract may be incorporated directly into food products or dried, cooled, frozen, or freeze-dried and then incorporated into the food products. Food product into which the glucosinolate extract may be incorporated include food supplements, drinks, shakes, baked goods, teas, soups, cereals, pills, tablets, salads, sandwiches, granolas, salad dressings, sauces, coffee, cheeses, yogurts, energy bars, and the like as well as mixtures thereof. Supplements include dietary supplements, nutritional supplements, herbal supplements, and the like. The food product may contain an effective amount of the glucosinolate extract, such as about 1 to about 100 mg per single serving of the food product. An effective amount of the glucosinolate extract may also be incorporated into pharmaceutical compositions, such as about 1 to about 100 mg per single dosage.

### BRIEF DESCRIPTION OF THE DRAWINGS

FIG. 1 provides the principal reactions for conversion of glucoraphanin to sulforaphane.

FIG. 2 provides a flowchart illustrating one embodiment of the process of the invention.

FIG. 3 provides a flowchart illustrating another embodiment of the process of the invention.

### DETAILED DESCRIPTION

The present process is both technically straightforward and attractive from a production cost standpoint. Indeed, the present process makes it possible to obtain glucosinolates from agricultural by-products and waste, particularly post-harvest glucosinolate-containing plants and discarded materials that would otherwise be discarded or plowed under in the fields, such as non-saleable produce, leaves, stems, seeds, and the like. The glucosinolates, particularly glucoraphanin, obtained from the process of the invention offer the health benefits of glucosinolates prepared from substantially more expensive processes.

As used herein, "chemoprotectants" and "chemoprotective compounds" refer to agents of plant origin that are effective for reducing the susceptibility of mammals to the toxic and neoplastic effects of carcinogens. Chemoprotectant "precursors" refer to agents which give rise to chemoprotectants by enzymatic and/or chemical means. Talalay, P. et al., J. Nutr., 131 (11 Supp.): 30275-30335 (2001). Examples of such chemoprotectant precursors include alkyl glucosinolates, such as glucoraphanin.

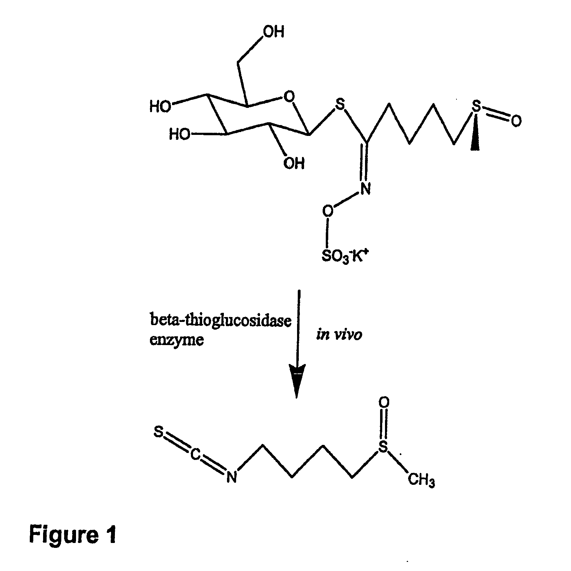

The thioglucosidic bonds of glucoraphanin are hydrolyzed *in vivo* by gut microflora into unstable glucosinolate aglycones, which undergo spontaneous rearrangement into isothiocyanates, such as sulforaphane, and other compounds, such as nitriles and thiocyanates. The conversion can also be catalyzed by beta-thioglucosidases, particularly myrosinase, which are found in glucosinolate-containing plants. The principal reactions are illustrated in FIG. 1.

As used herein, "effective amount" is an amount of additive which provides the desired effect or benefit upon consumption. Generally, about 1 to about 100 mg of the glucosinolates of the invention, particularly glucoraphanin, per single serving of the food product or about 1 to about 100 mg per single dosage of a pharmaceutical composition; higher amounts can be used if desired.

The term "plant materials" generally includes whole plants, leaves, plant tissue, sprouts, stems, seeds, florets, fruits, flowers, tubers, roots, the like, and mixtures thereof. While the amount of glucosinolates may vary from one part of the plant to another, from one type of plant to another, and may depend on the age of the plant, any combination of plant materials can be used so as to reduce the amount of sorting and preparation time prior to using the plant materials in the process of the invention although it is preferable to utilize the plant materials that contain desirable levels of the glucosinolates desired to be extracted.

Cruciferous vegetables have been identified as a good source of chemoprotectant precursor phytochemicals. Cruciferous vegetables include, but are not limited to, broccoli, kale, collard, curly kale, marrowstem kale, thousand head kale, Chinese kale, cauliflower, Portuguese kale, brussel sprouts, kohlrabi, Jersey kale, Chinese broccoli, rutabaga, mustard, daikon, horseradish, cabbage, savoy cabbage, Chinese cabbage, napa cabbage, broccoli rabe, arugula, watercress, cress, turnip, borecole, radish, and the like.

In a preferred aspect, broccoli plant materials are utilized. Particularly useful broccoli cultivars that may be used in the claimed method are Saga, DeCicco, Everest, Emerald City, Packman, Corvet, Dandy, Early, Emperor, Mariner, Green Comet, Green Valiant, Arcadia, Calabrese Caravel, Chancellor, Citation, Cruiser, Early Purple Sprouting Red Arrow, Eureka, Excelsior, Galleon, Ginga , Goliath, Green Duke, Greenblet, Italian Sprouting, Late Purple Sprouting, Late Winter Sprouting, White Star, Legend, Leprechaun, Marathon, Mariner, Minaret (Romanesco), Paragon, Patriot, Premium Crop, Rapine (Spring Raab), Rosalind, Salade (Fall Raab), Samurai, Shogun, Sprinter, Sultan, Taiko, Trixie, San Miguel, Arcadia, Gypsy, Everest, Patron, Southern Comet, Green Comet, Destiny, Climax and Pirate. However, many other broccoli cultivars are suitable.

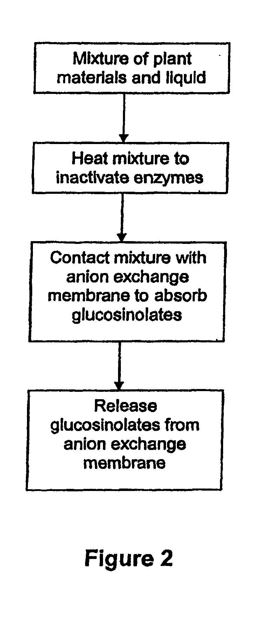

One embodiment of the present invention is illustrated in FIG. 2. The invention can be carried out in batch, semi-batch, semi-continuous, or continuous mode. The method of the invention generally comprises (1) providing a mixture of glucosinolate-containing plant material and liquid, (2) heating the mixture to inactivate enzymes in the plant material, (3) contacting the heat inactivated mixture with an anion exchange membrane whereby at least a portion of the glucosinolates are absorbed onto the anion exchange membrane, and (4) releasing the glucosinolates from the anion exchange membrane. The method may further comprise removing salt from the extracted glucosinolates.

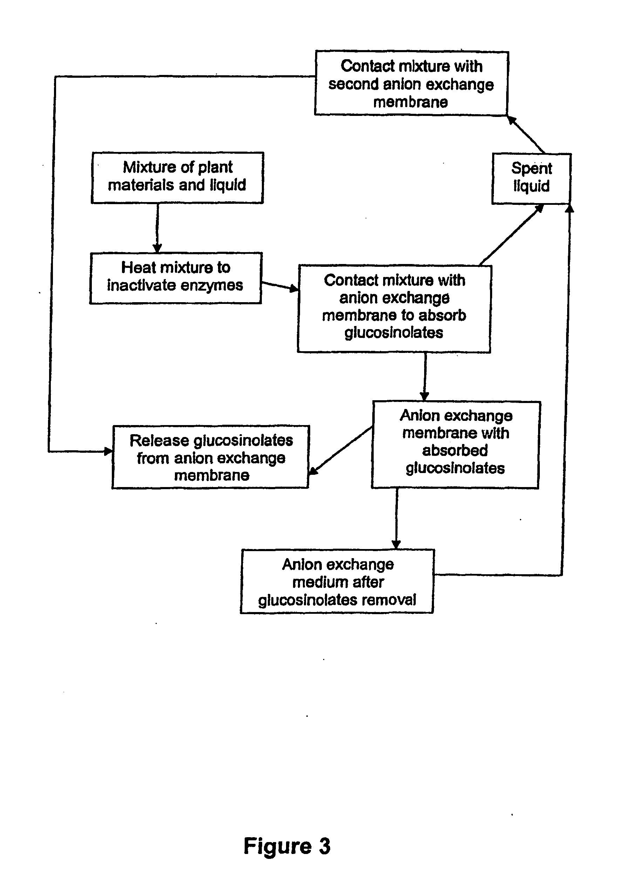

Another embodiment of the present invention is illustrated in FIG. 3. This method includes the following steps: (1) providing a mixture of glucosinolate-containing plant material and liquid; (2) heating the mixture to inactivate enzymes in the plant material; (3) contacting the heat inactivated mixture with an anion exchange membrane whereby at least a portion of the glucosinolates are absorbed onto the anion exchange membrane; (4) releasing the glucosinolates from the anion exchange membrane; (5) contacting the heat inactivated mixture with an anion exchange membrane whereby at least a portion of the glucosinolates are absorbed onto the anion exchange membrane; and (6) releasing the glucosinolates form the anion exchange membrane. The method may further comprise removing salt from the extracted glucosinolates.

While the method of the invention is suitable for agricultural by-products and waste plant materials, the plant materials should be relatively fresh and not show spoilage. Advantageously, the process of the invention is suitable for extremely crude samples, which is not commonly found in traditional processes for extracting glucosinolates from plant materials. Generally, the method of the invention does not require that the plant materials undergo any sort of purifying pretreatment (e.g., the plant materials do not need to be washed, filtered, centrifuged, or the like to remove soil, debris, or other insoluble components before use).

Generally, when post-harvest plant materials are used in the method of the invention, the plant materials should be processed by the method of the invention relatively quickly after harvesting, such as within a few hours. Glucosinolates remain intact in the plant materials until contacted by beta-thioglucosidase enzymes. The cell walls of the plant materials begin to break down post-harvest due to cellular senescence. Cellular breakdown brings the glucosinolates into contact with the enzymes, such as myrosinase, that convert glucosinolates into isothiocyanates. In order to prolong the period of time the plant materials can be used post-harvest, it may be desirable to preserve the glucosinolate-containing plant materials, such as by freezing, or drying, such as by air drying, freeze drying, vacuum drying, oven drying, or the like. Preserving the glucosinolate-containing plant materials serves to reduce the amount of conversion of glucosinolates to isothiocyanates by beta-thioglucosidases in the plant material as well as to reduce the activity of spoilage microorganisms. Generally, however, it is preferred that the materials be used soon after harvesting.

In another important aspect, damage to the glucosinolate-containing plant materials should be minimized until immediately before use in the method of the invention (i.e., within a few hours). Damage to the plant materials, such as by chewing, crushing, or cutting, also triggers the conversion of glucosinolates to isothiocyanates by beta-thioglucoisdases. Therefore, damage to the glucosinolate-containing plant materials should be minimized prior to utilizing the plant materials in the method of the invention.

immediately prior to use in the method of the invention, such as about 1 to about 2 hours before use, the glucosinolate-containing plant materials should be treated to disrupt the cellular structure in order to facilitate the release of glucosinolates. Suitable treatment steps include, but are not limited to, pulverizing, crushing, chopping, puréeing, the like, or a combination thereof. If the plant materials include seeds, the seeds can be pulverized and defatted prior to use. In one aspect, the seeds may be defatted using known defatting procedures, such as described in West, L., J. Agric. Food Chem., 52: 916-926 (2004).

The plant materials should then be heated for a time and temperature sufficient to inactivate enzymes in the glucosinolate-containing plant material. Generally, the plant materials are heated to 60°C to 110°C for at least about five minutes. The heat treatment may be by any conventional means, such as by boiling, steaming, microwaving. Preferably, the heat treatment step inactivates beta-thioglucosidase enzymes, preferably myrosinase, present in the plant materials in order to prevent the conversion of glucosinolates to isothiocyanates. Advantageously, the heat treatment also inactivates microbes in the mixture. It has also been found that the heat treatment improves the extraction of glucosinolates from the plant materials by increasing solubility and increasing water penetration into the plant materials. Generally, if boiling is used as the heat treatment, the amount of liquid is not critical as long as there is sufficient liquid to thoroughly and evenly heat the plant materials.

Depending on the method of heating selected, it may be necessary to increase the volume of liquid in the mixture following the heat treatment step. Generally, the liquid is water but may be water containing an organic solvent, such as ethyl alcohol. Preferably, the liquid is water. The amount of liquid in the mixture should be sufficient to make a stirrabie slurry.

The heat inactivated mixture is then contacted with an anion exchange membrane to extract glucosinolates from the heat inactivated mixture. Preferably, the anion exchange membrane is a strong base type membrane but weak base membranes may be used, if desired. Depending on the type of membrane selected, the membrane may require pre-treatment, such as soaking to "swell" the membrane. Preferably, the anion exchange membrane is formed as a sheet and is immersed in the heat inactivated mixture. It is generally preferred to circulate the heat inactivated mixture during the extraction step, such as by stirring, mixing, or agitating by any convenient means. The anion exchange membrane contacts the heat inactivated mixture for 4 to 24 hours to allow glucosinolates to absorb to the anion exchange membrane. The temperature during extraction is sufficiently low to decrease the risk of microbial outgrowth during the extraction steep, i.e. at a temperature of 2 to 7°C. Suitable anion exchange membranes include strongly basic membranes, such as Electropure Excellion anion exchange membrane i 200 from Snowpure, LLC in San Clemente, CA. The surface of the membrane has "locked in place" groups having a positive charge and an appropriate counter ion, such as chloride. Glucosinolates have a stronger negative charge and take the place of the chloride ion, thus binding the glucosinolates to the membrane.

Once the glucosinolates have been absorbed to the membrane, it is preferable to wash the anion exchange membrane with de-ionized water to remove debris. The anion exchange membrane having bound glucosinolates is then contacted with an aqueous liquid containing salts, such as KCI, NaCI, or other food-grade inorganic salts, to facilitate the release of bound glucosinolates from the anion exchange membrane into the aqueous liquid. Preferably, the aqueous liquid contains 1 M KCI. Generally, the amount of liquid is not critical as long as there is sufficient liquid to cover the membrane. Generally, about 4 to about 5 hours is sufficient time to release the glucosinolates from the membrane. Generally, at least about 80 percent of the glucosinolates are removed from the membrane. Again, the temperature should be sufficiently low as to reduce the risk of microbial outgrowth during the release step, such as a temperature of 2 to 7°C.

Generally it is preferred to repeat the extraction and release steps at least once to maximize the amount of glucosinolates removed from the heat inactivated mixture. Preferably, the same anion exchange membrane used in the previous extraction and release steps is used although a new anion exchange membrane may be used if desired.

The glucosinolates released into the aqueous liquid may be further treated if desired. Preferably, the aqueous medium containing the glucosinolates is treated to remove salt. Salt is removed because salt is generally undesired in the final product. Generally, salt can be removed from the aqueous medium by any conventional technique including, for example, dialyzing against deionized water using one or more cellulose ester membrane. Preferably, salt is removed by dialyzing against de-ionized water for 12 to 24 hours at 2 to 7°C using one or more 100 molecular weight cutoff (MWCO) cellulose ester membranes.

The glucosinolates released into the aqueous liquid may be further processed into a variety of forms, if desired. For example, the glucosinolates may be dried, such as by spray drying, freeze drying, vacuum drying, or the like, to form a dried glucosinolate extract. Generally, the resulting extract can then be dried for a time sufficient to reduce the water content of the extract to less than about 10 percent, preferably to less than about 5 percent, to form a dried glucosinolate extract. Generally, the extract may be dried using any known method, such as, but not limited to, freeze drying, spray drying, vacuum drying, and the like.

The glucosinolate extract may also be further processed by cooling or freezing, or may be subjected to membrane processing, chromatographic processing, or dialysis to remove unwanted anionic substances that were bound to and were released from the anion exchange membrane, such as to form a glucosinolate isolate or purified product.

The glucosinolate extract may also have introduced optional ingredients or components, such as, for example, flavorants, nutrients, vitamins, colorants, nutraceutical additives, antioxidants, probiotics, and the like, so long as the optional ingredients do not adversely affect the stability in a significant manner. In particular, the presence and amount of such optional ingredients can, of course, vary considerably depending on the product in which the extract is incorporated.

The glucosinolate extract may be included in a variety of products, including food products and pharmaceuticals. The glucosinolate extract may also be used as a food fortificant. Food product into which the glucosinolate extract may be incorporated include food supplements, drinks, shakes, baked goods, teas, soups, cereals, pills, tablets, salads, sandwiches, granolas, salad dressings, sauces, coffee, cheeses, yogurts, energy bars, and the like as well as mixtures thereof. Supplements include dietary supplements, nutritional supplements, herbal supplements, or the like.
In this aspect, the food product may contain an effective amount of glucosinolate extract, such as about 1 to about 100 mg per single serving of the food product. An effective amount of the glucosinolate extract may also be incorporated into pharmaceutical compositions, such as about 1 to about 100 mg. Of course, higher amounts can be included if desired.

Following the process of the invention, the spent plant materials used in the invention may be recycled and used as feed material for animals. Generally, the spent plant material can be separated from the aqueous liquid by filtration, centrifugation, decanting, or the like, to provide plant materials suitable for animal feed. The spent plant materials are uniquely useful as feed material because the materials no longer contain glucosinolates. It is generally desired that plant materials used for animal feed have low levels of glucosinolates because glucosinolate-containing plant materials have been found to have undesired effects on animals when used as a primary food source.

The following examples are intended to illustrate the invention and not to limit it. Unless otherwise stated, all percentages, parts, and ratios are by weight. All references (including publications, patents, patent applications, patent publications, and the like) cited in the present specification are hereby incorporated by reference.

### EXAMPLES

### Example 1: Glucosinolates from broccoli seeds

Fifteen grams of broccoli seeds were defatted by hexane extraction and pulverized to pass through a #18 seive. The pulverized and defatted broccoli seeds were added to 1500 ml of de-ionized water to form a mixture. The mixture was boiled for 5 minutes to inactivate the beta-thioglucosidase enzymes in the seeds. The total volume of the mixture was then brought to 4500 ml with de-ionized water. One square foot of Electropure Excellion anion exchange membrane heterogeneous strong base, Type 1, Model I-200 (Snowpure, LLC in San Clemente, CA), was suspended in the continuously stirred mixture held at 5°C for 24 hours. After extraction, the anion exchange membrane was briefly washed with deionized water and the membrane was placed in a stirred tank containing 4500 ml of 1 N KCI at 5°C for 4 hours to release the glucosinolates from the anion exchange membrane. The glucoraphanin concentration of the aqueous medium was measured by HPLC. Extraction over 24 hours resulted in 58 percent removal of total available glucoraphanin. 1 N KCI released essentially all bound glucoraphanin in 4 hours.

To maximize the yield of glucosinolates, both the extraction and release steps are repeated. The same anion exchange membrane was returned to the extraction tank for an additional 18 hours. The anion exchange membrane was then returned to the same 1 N KCI tank for four hours. The total removal of glucoraphanin was increased to 81 percent by repeating the process as described. A 4 hour treatment in the same 1 N KCl tank again released essentially all of the bound glucoraphanin.

Following the extraction and release steps, salt was removed by dialyzing against de-ionized water overnight at 5°C using 100 molecular weight cutoff (MWCO) cellulose ester membranes.

### Example 2: Glucosinolates from broccoli florets

The process of Example 1 was repeated using 150 g dried and pulverized broccoli florets. The broccoli florets were pulverized to pass through a #18 sieve. The process resulted in greater than 80 percent recovery of glucosinolates.

### Example 3: Glucosinolates from plants

The process of Example 1 was repeated using 1 kg freshly harvested broccoli leaves and stems. The broccoli leaves and stems were steamed to inactivate the beta-thioglucosidase enzymes for 20 minutes. The broccoli leaves and stems were then puréed. The process resulted in recovery of glucosinolates similar to that of Example 1.

## Claims

1. A method of extracting glucosinolates from glucosinolate-containing plant material, said method comprising:
(i) providing a mixture of glucosinolate-containing plant material and liquid;
(ii) heating the mixture for a time and temperature sufficient to inactivate enzymes in the glucosinolate-containing plant material to form a heat inactivated mixture;
(iii) contacting the heat inactivated mixture with an anion exchange membrane for 4 to 24 hours and a temperature of 2 to 7°C, whereby at least a portion of the glucosinolates are absorbed onto the anion exchange membrane; and
(iv) contacting the anion exchange membrane having absorbed glucosinolates with an aqueous solution for a time sufficient to release the extracted glucosinolates from the anion exchange membrane to form an aqueous media containing the glucosinolates.

2. The method of claim 1 wherein the glucosinolate-containing plant material is selected from the group consisting of broccoli, kale, collard, curly kale, marrowstem kale, thousand head kale, Chinese kale, cauliflower, Portuguese kale, brussel sprouts, kohlrabi, Jersey kale, Chinese broccoli, rutabaga, mustard, daikon, horseradish, cabbage, savoy cabbage, Chinese cabbage, napa cabbage, broccoli rabe, arugula, watercress, cress, turnip, borecole, radish, the like, and mixtures thereof.

3. The method of claim 1 or 2 wherein the glucosinolate-containing plant material comprises at least one of the group consisting of florets, seeds, leaves, sprouts, and stems.

4. The method of any one of claims 1 to 3 further comprising pre-treating the glucosinolate-containing plant material in step (i) by at least one of the group consisting of dehydrating, defatting, freezing, pulverizing, crushing, chopping, purèeing, or a combination thereof.

5. The method of any one of claims 1 to 4 wherein the heating step is at a temperature of 60 to 110°C for 5 to 15 minutes.

6. The method of any one of claims 1 to 5 wherein the extracted glucosinolates are glucoraphanin.

7. The method of any one of claims 1 to 6 further comprising repeating steps (iii) and (iv) after step (iv).

8. The method of any one of claims 1 to 7 further comprising removing salt from the extracted glucosinolates.

## Patentansprüche

1. Verfahren zum Extrahieren von Glukosinolaten von glukosinolathaltigem Planzenmaterial, wobei das genannte Verfahren Folgendes beinhaltet:
(i) Bereitstellen eines Gemischs von glukosinolathaltigem Pflanzenmaterial und Flüssigkeit;
(ii) Erhitzen des Gemischs für eine Zeit und bei einer Temperatur, die ausreichen, um Enzyme in dem glukosinolathaltigen Pflanzenmaterial zu inaktivierten, um ein hitzeinaktiviertes Gemisch zu bilden;
(iii) Inkontaktbringen des hitzeinaktivierten Gemischs mit einer Anionenaustauschmembran für 4 bis 24 Stunden und bei einer Temperatur von 2 bis 7°C, so dass wenigstens ein Teil der Glukosinolate auf die Anionenaustauschmembran absorbiert wird; und
(iv) Inkontaktbringen der absorbierte Glukosinolate aufweisenden Anionenaustauschmembran mit einer wässrigen Lösung für eine Zeit, die ausreicht, um die extrahierten Glukosinolate von der Anionenaustauschmembran freizusetzen, um ein die Glukosinolate enthaltendes wässriges Medium zu bilden.

2. Verfahren nach Anspruch 1, wobei das glukosinolathaltige Pflanzenmaterial ausgewählt wird aus der Gruppe bestehend aus Brokkoli, Grünkohl, Blattkohl, Krauskohl, Futterkohl, Tausendkopfkohl, Chinakohl, Blumenkohl, portugiesischem Grünkohl, Rosenkohl, Kohlrabi, Spazierstockkohl, chinesischem Brokkoli, Kohlrübe, Senf, Daikon, Meerrettich, Kraut, Wirsingkohl, chinesische Kohl, Selleriekohl, Wildbrokkoli, Rukola, Wasserkresse, Kresse, Kohlrübe, Federkohl, Rettich und dergleichen und Gemischen davon.

3. Verfahren nach Anspruch 1 oder 2, wobei das glukosinolathaltige Pflanzenmaterial wenigstens ein Mitglied der Gruppe bestehend aus Blüten, Samen, Blättern, Sprossen und Stielen umfasst.

4. Verfahren nach einem der Ansprüche 1 bis 3, das ferner das Vorbehandeln des glukosinolathaltigen Pflanzenmaterials in Schritt (i) mit wenigstens einem Mitglied der Gruppe bestehend aus Dehydrieren, Entfetten, Frieren, Pulverisieren, Zermahlen, Zerhacken, Pürieren oder einer Kombination davon beinhaltet.

5. Verfahren nach einem der Ansprüche 1 bis 4, wobei der Erhitzungsschritt bei einer Temperatur von 60 bis 110°C für 5 bis 15 Minuten erfolgt.

6. Verfahren nach eine der Ansprüche 1 bis 5, wobei die extrahierten Glukosinolate Glukoraphanin sind.

7. Verfahren nach einem der Ansprüche 1 bis 6, das ferner das Wiederholen der Schritte (iii) und (iv) nach Schritt (iv) beinhaltet.

8. Vorfahren nach eine der sprüche 1 bis 7, das ferner das Entfernen von Salz aus den extrahierten Glukosinolaten beinhaltet.

## Revendications

1. Procédé d'extraction de glucosinolates d'une substance végétale contenant des glucosinolates, ledit procédé comprenant :
(i) la fourniture d'un mélange de substance végétale contenant des glucosinolates et de liquide ;
(ii) le chauffage du mélange pendant une durée et à une température suffisantes pour inactiver les enzymes dans la substance végétale contenant des glucosinolates afin de former un mélange inactivé par la chaleur ;
(iii) la mise en contact du mélange inactivé par la chaleur avec une membrane d'échange anionique pendant 4 à 24 heures et à une température de 2 à 7 °C, par laquelle au moins une partie des glucosinolates est absorbée sur la membrane d'échange anionique ; et
(iv) la mise en contact de la membrane d'échange anionique à glucosinolates absorbés avec une solution aqueuse pendant une durée suffisante pour libérer les glucosinolates extraits de la membrane d'échange anionique afin de former un milieu aqueux contenant les glucosinolates.

2. Procédé selon la revendication 1, dans lequel la substance végétale contenant des glucosinolates est sélectionnée dans le groupe consistant en brocoli, chou fourrager, chou vert, chou vert frisé, chou moellier, mille-têtes, borécole chinois, chou-fleur, chou cabus, choux de Bruxelles, chou-rave, chou de Jersey, brocoli chinois, rutabaga, moutarde, daikon, raifort, chou, chou de Savoie, chou de Chine, chou napa, rapini, roquette, cresson de fontaine, cresson, navet, borécole, radis, végétaux assimilés et leurs mélanges.

3. Procédé selon la revendication 1 ou 2, dans lequel la substance végétale contenant des glucosinolates comprend au moins l'un du groupe consistant en fleurons, graines, feuilles, germes et tiges.

4. Procédé selon l'une quelconque des revendications 1 à 3, comprenant en outre le prétraitement de la substance végétale contenant des glucosinolates à l'étape (i) par au moins l'une des opérations du groupe consistant en déshydratation, dégraissage, congélation, pulvérisation, broyage, hachage, réduction en purée ou une combinaison de celles-ci.

5. Procédé selon l'une quelconque des revendications 1 à 4, dans lequel l'étape de chauffage est effectuée à une température de 60 à 110 °C pendant 5 à 15 minutes.

6. Procédé selon l'une quelconque des revendications 1 à 5, dans lequel les glucosinolates extraits sont de la glucoraphanine.

7. Procédé selon l'une quelconque des revendications 1 à 6, comprenant en outre la répétition des étapes (iii) et (iv) l'étape (iv).

8. Procédé l'une quelconque des revendications 1 à 7, comprenant en outre l'élimination du sel des glucosinolates extraits.
